# EUROPEAN PATENT SPECIFICATION

(11) **EP 1 485 626 B1**
(45) Date of publication and mention of the grant of the patent: **06.06.2007**
(21) Application number: 03742951.1
(22) Date of filing: 21.02.2003
(51) Int. Cl.: F16B 23/00, A61B 17/86, A61B 17/88, F16B 35/04

(54) **THREADED DEVICE WITH IMPROVED RESISTANCE AGAINST TORSION-CAUSED BREAKAGE**
GEWINDEVORRICHTUNG MIT VERBESSERTEM WIDERSTAND GEGEN DURCH TORSION VERURSACHTES BRECHEN
DISPOSITIF FILETE A RESISTANCE AMELIOREE A LA RUPTURE CAUSEE PAR LA TORSION

(30) Priority: 26.02.2002 EP 02004444
(43) Date of publication of application: 15.12.2004
(73) Proprietor: Degima Medizinprodukte GmbH, 25421 Pinneberg (DE)
(72) Inventor: TSCHAKALOFF, Alexander, 25421 Pinneberg (DE); VON OEPEN, Randolf, 72074 Tübingen (DE)
(74) Representative: Hoefer, Theodor
(86) International application number: PCT/EP2003/001793
(87) International publication number: WO 2003/072962

(56) References cited:
- EP-A- 0 502 698
- GB-A- 2 194 827
- US-A- 5 019 079
- US-A- 5 169 400
- US-B1- 6 319 270

## Description

### Background of the Invention

The present invention concerns a device for inserting into a tissue or a structure, according to the preamble part of claim 1.

Such a device is known from US-A-5 019 079, US-B1-6 319 270 or GB-A-2 194 827.

EP-A-0502698 discloses a bio absorbal interference bone fixation screw and US-A-5169400 discloses a bone screw made of resorbing plastic material.

For occasions where two pieces have to be connected or reconnected to each other often threaded devices like screws or pins are used. They are inserted through both pieces by screwing into a predrilled hole or by self-tapping a hole during insertion thereby fixing the pieces together. Other types of threaded devices are used to attach something. In the medical field, screws or pins are used to fix the pieces of a broken bone together so that they could reconnect. In other cases threaded devices are used to attach a fixation plate to the bone or a suture thread to the bone for connecting soft tissue or ligaments.

Threaded devices like screws are made from all suitable materials dependent on their application. Mainly they are constructed of metal or any kind of plastics, in the medical field also from biodegradable materials.

For insertion of threaded devices a force is applied on the top so that the device is turning around its longitudinal axis, thereby getting screwed and anchored into the material below. If the material is rigid or the device gets stuck it could happen that the device could not stand the applied force and breaks due to torsion along the longitudinal axis. In most cases the screw breaks at the transition between the part including the opening for the screwdriver and the part that is already screwed into the material. Especially with regard to screws made from plastics this is a well-known problem.

### Description of the Invention

It is an object of the present invention to have a threaded device that could be inserted without the risk of breaking due to torsion, especially for devices made from plastics. In addition it is desirable to have a threaded device that could be screwed into a material comprising a different elasticity of torsion than the material of the device. The present invention concerns a device according to the preamble part of claim 1 as well as a method according to claim 31.

The solution of this object is achieved by the features of claims 1 and 31, respectively.

The threaded device of the present invention overcomes the disadvantage described above by providing openings located distant from the centre of the body, preferably as far as possible at the outer margin of the screw body, for receiving an insertion instrument.

### Brief Descriptions of the Drawings

FIG. 1 is a plan view of a device according to the present invention showing the proximal head portion, the thread portion of the device body and the distal tip.
FIG. 2 is a plan view of the device illustrated in FIG. 1, but turned 90° around the longitudinal axis of the device, showing a passageway in the thread portion parallel to the longitudinal axis of the device and the proximal opening of the passageway at the proximal end of the device.
FIG. 3 is a cross-sectional view of the device illustrated in FIG. 1 showing two passageways on each side of the middle part of the device including the corresponding openings at the proximal end.
FIG. 4 is a view from the proximal end of the device illustrated in FIG. 1 showing the proximal openings for the use of a corresponding insertion instrument.
FIG. 5 (A to F) shows possible variations of shape and number of the proximal openings of a device according to the present invention.
FIG. 6 is a cross-sectional view of a threaded device according to the present invention comprising threaded and non-threaded portions of the body.
FIG. 7 is a cross-sectional view of a threaded device according to the present invention comprising different thread pitches and tapering of the diameter.
FIG. 8 is an illustration of the relation between radius r and force F for constant torque M (for different proximal openings).

Detailed Description of the Drawings and the Invention

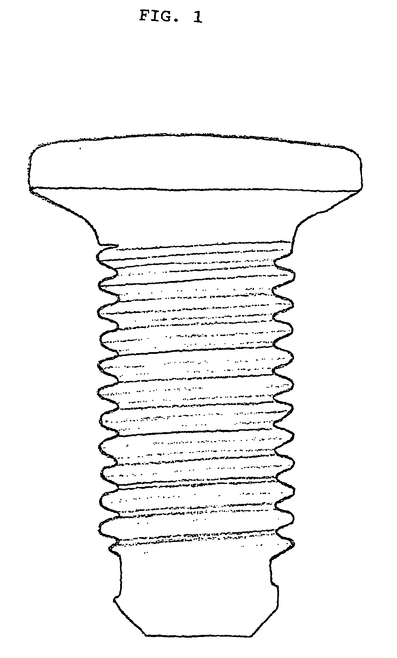

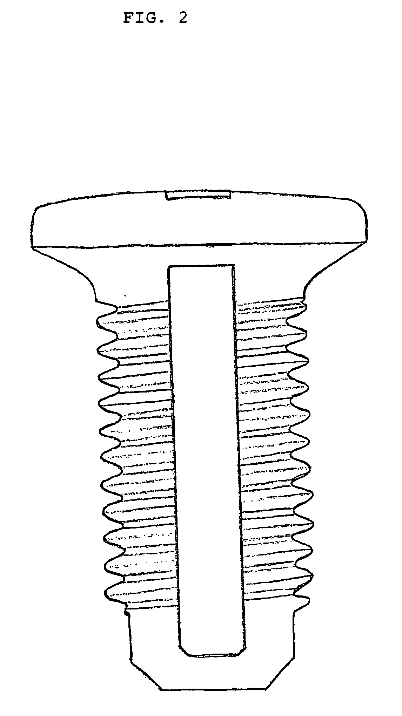

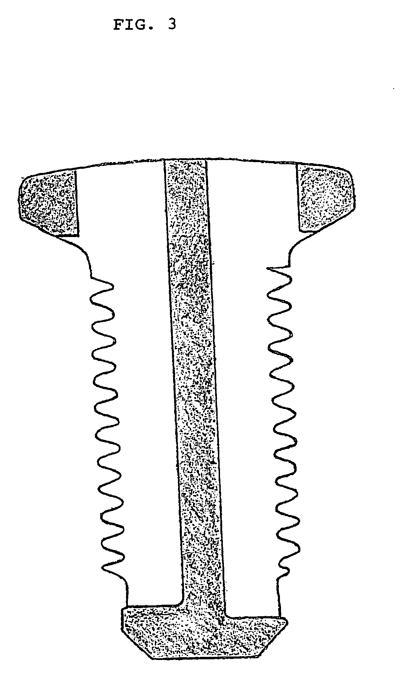

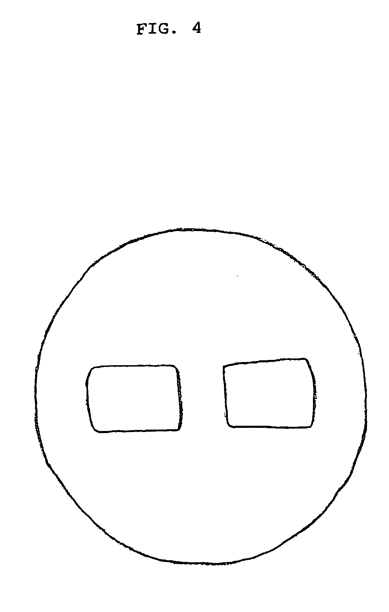

FiGs. 1 to 4 illustrate one example for a threaded device according to the present invention. In FIG. 1 the screw is shown from one side, in FIG. 2 the same screw is shown after turning the screw for 90° around the longitudinal axis. The screw comprises a proximal portion and a distal portion, a portion positioned to lie between the proximal and distal portion including exterior screw threads for inserting and retaining said body in tissue or other material. In addition this screw comprises two passageways extending parallel to the longitudinal axis. In FIG. 2 one passageway is illustrated extending in the middle of the body, FIG. 3 shows the two passageways extending through the body of the screw and in FIG. 4 the two openings of the passageways at the proximal end are shown. The proximal openings of the passageways are designed for receiving an insertion instrument.

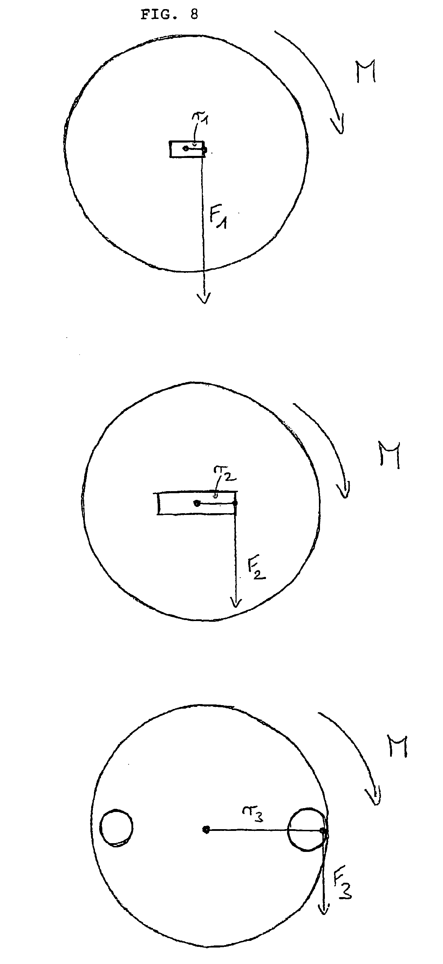

The torque *M* of a turning body is defined as *M* = *r* × *F .* If *M* is constant it is valid for different *r*₁, *r*₂ *, r*₃ ... : *M* = *r₁* × *F*₁ = *r*₂ × *F*₂ = *r*₃ × *F*₃*,* (*r* - radius, *F* - force). For increasing values of *r* like *r*₁〈*r₂〈r*₃ the force will behave like *F*₁*〉F*₂*〉F*₃*.* Accordingly the application of force on a threaded, turning device is smaller the more distant the point of application of the force is from the central point (see FIG. 8). If the passageways are placed as far as possible from the centre of the device, less force is needed for turning the device. Subsequently less force is needed for insertion of the threaded device and less torsion is produced. This finally reduces the risk of breakage of the body due to the tension. This is even more advantageous for materials comprising a low modulus of elasticity such as polymers. Within the scope of this invention it is therefore desirable to place the passageways used for inserting the device as far as possible from the centre point of the threaded body. In the example shown in FiGs. 1 to 4 the passageways are placed so close to the margins of the threaded body of the device that they comprise openings directed to the side (see FIG. 3).

The body of the device described here could be basically cylindrical or tapered to satisfy the needs of the various applications within the medical field. The passageway(s) could be tapered or not, again dependent on the need of the various applications. It is also understood that the passageway(s) is (are) extending through a majority of the body. It is further understood that at least one passageway of the device is extending through the majority of the body of the device.

As shown in FIG. 1 to 3 the threaded device could comprise a proximal non-threaded portion. This portion could be flat or curved at the proximal end, it could comprise means for fixing threads, plates or any necessary item. If the device does not comprise a proximal non-threaded portion it could be used as a threaded rod.

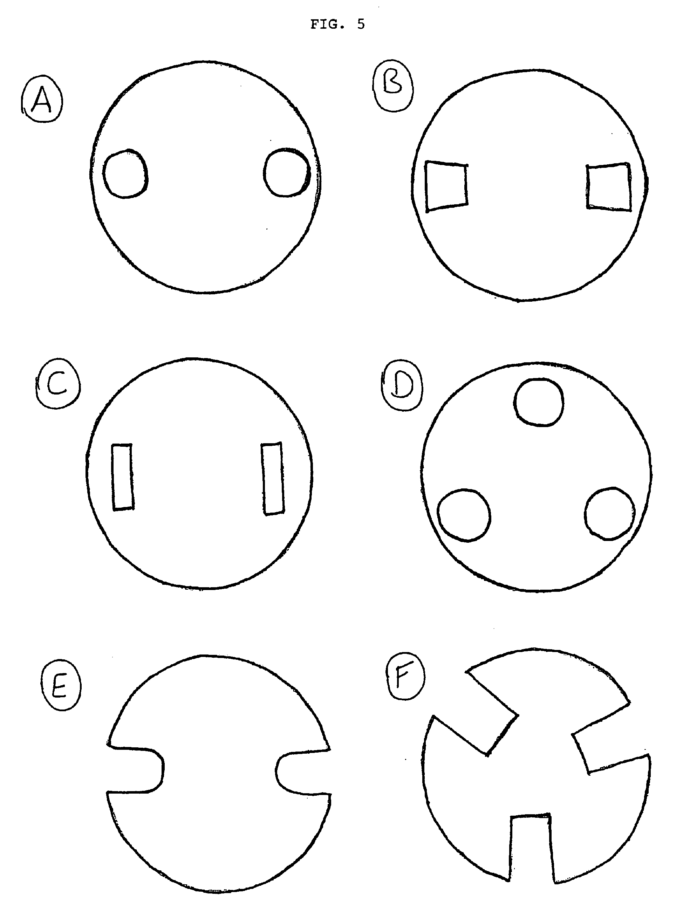

The screw could comprise two openings as shown in FIG. 4, it could also comprise more openings. Finally it could also comprise only one asymmetric located opening plus an additional central fixed point. All these openings could have every suitable shape, in particular they could be shaped like a slit, a circle, a square or a rectangle (see FIG. 5). The openings could be surrounded by material as illustrated in FIG. 5 A to D or they could extend until the end of the proximal portion (see FIG. 5 E and F). If the openings are surrounded by material the proximal portion comprises more stability against the screwing force and the resulting torsion.

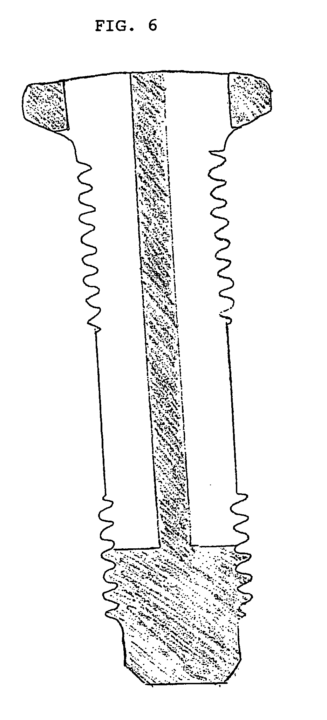

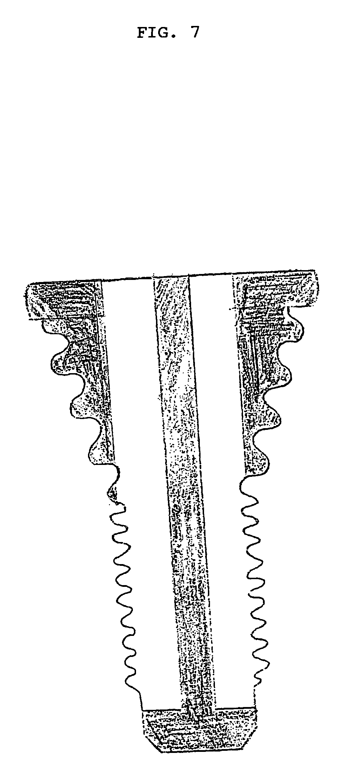

The threaded devices could either be self-tapping or could be screwed into pre-drilled holes. In addition to the threaded portion they could also comprises at least one non-threaded portion for special applications (see FIG. 6). The threaded portion could also comprise different thread pitches and could be tapered different in different parts (see FIG. 7). A threaded device as illustrated in FIG. 7 could be used as a bone-screw where the two bones are pulled together during insertion of the device.

Threaded devices according to the present invention could be used for all possible applications, in particular for applications with need of threaded devices made from polymers or from biodegradable polymers such as in the medical field including screws, pins, anchors and suture anchors. The threaded device according to the present invention could also comprise additional passageways for receiving a suture thread. In the medical field such a device could be used as a suture anchor to connect a soft tissue or a ligament to bone.

It might be necessary to design a special insertion instrument to fit exactly to the special proximal openings and the corresponding passageways extending through the body of the device. Such an instrument could also work as an thread cutter if the passageways are open at the side as shown in FIG. 3. On the other hand it could be possible to adapt the proximal openings to normal, commercial available screwdrivers. The material of the screw and/or the screwdriver could be chosen in a way the screw attaches very easy to the screwdriver via friction. The surgeon prefers this characteristic since it is very helpful if the comparable tiny screws are somehow attaching to the screwdriver.

As shown in FIG. 3 the passageways extend through the screw body almost to the distal tip. They could also extend only through a proximal part or they could extend through the whole body comprising also distal openings if necessary. Preferably the passageways advance through a long portion of the screw body (as in FIG. 3) since the insertion instrument is then not only applying force to the proximal part but to the whole device, which reduces the risk of a breakage between proximal part and threaded body.

The device may be made from any suitable material. If the device should be applied in the medical area any biocompatible material is preferred to minimise anti-inflammatory response. It could be manufactured from titanium, magnesium, thallium, stainless steel, Nitinol or any other suitable metals or any suitable polymer. Advantageous is also the use of biodegradable polymers, if the device is implanted into the body. The device is then only needed for a limited time, e.g. until the pieces of a bone are reconnected. Preferred materials include polysaccharides, polylactides, polyglycolids, polydiaxanoles or other suitable biodegradable polymers or according copolymers or blends. Degradation of implants made from biodegradable polymers often leads to irritation of the surrounding tissue due to the release of substances like lactic acids. To limit this risk of irritation special compounds could be incorporated into the device material like antibiotics, immunosuppressiva, anti-inflammatory drugs or other suitable pharmaceuticals. If a biodegradable device is inserted into bone, incorporated bone growth factors could help to promote the ingrowth of the bone into the cavity left to replace the implant. A mixture of different compounds might be useful for special applications.

To change the characteristics of the device, e.g. the coefficient of friction, a coating could cover the device. When the surface has less friction, it is easier to insert the device. Implanted devices could also be covered by a biodegradable coating, eventually containing one or more compounds selected from the group of antibiotics, immunosuppressiva, anti-inflammatory drugs, bone growth factors or other suitable pharmaceutical to improve the acceptance of the implant.

For the manufacture of devices according to the present invention every suitable method could be applied, dependent on the material. Devices made from polymers could be manufactured by injection moulding, by extrusion combined with mechanical finish, by producing a casted block, which is subsequently machined, or by using a block produced by polymerisation, which is subsequently machined. The manufacture process of injection moulding could be controlled in a way to result in an orientation of the polymer, so that the device will relax after implantation and therefore will foreshorten and swell. This will then lead to additional anchoring of the device, e.g. into the bone. The density of the outer layer of the device could be influenced by different injection parameters as well, which results in different degradation times. To manufacture a device with incorporated compounds as discussed above devices could be incubated in a solution of the compound(s), so that there will be loaded a certain amount of compound into the device. Dependent on its characteristics the compound could be added to the polymer solution or the polymer resin prior to manufacturing of the device.

Another aspect of the invention is a method of inserting a threaded device into a tissue or structure that comprises the following steps. First, a device is provided that comprises a body having a proximal and distal end, a threaded portion adjacent to and positioned to lie between the proximal and distal portion and at least one passageway extending through the body, said passageway having at least one proximal opening for receiving an insertion instrument. The passageway is thereby orientated such that the torsion is reduced during insertion of the device by the insertion instrument. Second, a hole is eventually drilled into the tissue or structure. Finally the device is inserted into the tissue or structure via an insertion instrument with reduced torsion of the device.

It will be understood that the above-described device is only one example that illustrates the principle of the invention. Various modifications can be made by those skilled in the art without departing from the scope of this invention, as defined by the appended claims.

## Claims

1. A device for inserting into a tissue or a structure, comprising:
- a body having a proximal end, a distal end and a centre; and
- a threaded portion adjacent to and positioned to lie between the proximal and distal portion including exterior screw threads for inserting and retaining said body in the tissue or the structure, **characterized in**
- at least one passageway extending through at least a part of the body, said passageway having at least one proximal opening for receiving an insertion instrument; the passageway being placed distant to the centre of the body such that the torsion during insertion of the device is reduced, wherein the at least one passageway is extending through a majority of the body.

2. The device of claim 1, wherein the body of the device is basically cylindrical.

3. The device of claim 1, wherein the body of the device is tapered.

4. The device of claim 1, wherein the at least one passageway is not tapering.

5. The device of claim 1, wherein the at least one passageway is tapering.

6. The device of claim 1, wherein the body of the device comprises at least one non-threaded portion.

7. The device of claim 1, wherein the device is constructed of a biocompatible material.

8. The device of claims 1 to 7, wherein the device is made from titanium, magnesium, thallium, stainless steel, nitinol or any other suitable metals.

9. The device of claims 1 to 7, wherein the device is made from a polymer.

10. he device of claim 9, wherein the device is made from a biodegradable polymer.

11. The device of claim 10, wherein the device is made from polysaccharide, polylactide, polyglycolid, polydiaxanole or other suitable biodegradable polymers or according copolymers or blends.

12. The device of claim 10, wherein the material of the device has incorporated one or more compounds selected from the group of antibiotics, immunosuppressiva, anti-inflammatory drugs, bone growth factors or other suitable pharmaceuticals.

13. The device of claims 1 to 7, wherein the device is covered with a biodegradable coating.

14. The device of claim 13, wherein said coating includes one or more compounds selected from the group of antibiotics, immunosuppressiva, anti-inflammatory drugs, bone growth factors or other suitable pharmaceuticals.

15. The device of claims 1 to 7, wherein the device is covered with a coating comprising a low coefficient of friction.

16. The device of claims 1 to 7, wherein the proximal end comprises a nonthreaded proximal portion.

17. The device of claims 1 to 7, wherein said exterior device threads are seif-tapping.

18. The device of claims 1 to 7, wherein said device comprises means for receiving a thread.

19. The device of claim 1, wherein the at least one proximal opening is shaped like a slit, a circle or a square.

20. The device of claim 1, wherein the device is attaching to the instrument via friction.

21. An Instrument for inserting a device according to claim 1 into a tissue or structure comprising means for attaching to the at least one proximal opening and/or the at least one passageway of the device.

22. The Instrument according to claim 21, wherein the Instrument is working as a thread cutter.

23. A process to manufacture a device according to claim 9 or 10 using injection moulding.

24. A process to manufacture a device according to claim 9 or 10 using extrusion combined with mechanical finish.

25. A process to manufacture a device according to claim 9 or 10 using a casted block, which is subsequently machined.

26. A process to manufacture a device according to claim 9 or 10 using a block produced by polymerisation, which is subsequently machined.

27. A process to manufacture a device according to claim 12 or 14, wherein the compound is incorporated into the device by incubating the device with a solution of the compound.

28. A process to manufacture a device according to claim 12 or 14, wherein the compound is added to the polymer prior to the manufacturing process of the device.

29. The process of claim 23, wherein the manufacturing process leads to an orientation of the polymer, so that the device will relax after implantation and therefore will foreshorten and swell, which will lead to additional anchoring of the device into the tissue or structure.

30. A method of inserting the threaded device of claims 1-20 into a tissue or structure, comprising the steps of:
- providing a device comprising a body having a proximal and distal end, a threaded portion adjacent to and positioned to lie between the proximal and distal portion and at least one passageway extending through a majority of the body, said passageway having at least one proximal opening for receiving an insertion instrument; the passageway being non-centered such that the torsion is reduced during insertion of the device by the insertion instrument,
- eventually drilling a hole into the tissue or structure,
- inserting the device into the tissue or structure via an insertion instrument with reduced torsion of the device.

## Patentansprüche

1. Vorrichtung zum Einführen in ein Gewebe oder eine Struktur, umfassend:
- einen Körper mit einem proximalen Ende, einem distalen Ende und einer Mitte; und
- einen Gewindebereich, angrenzend an den proximalen und distalen Bereich und angeordnet, um zwischen diesen zu liegen, mit Außenschraubengewinden zum Einführen und Halten des Körpers in dem Gewebe oder der Struktur, **gekennzeichnet durch**
- zumindest einen Durchgang, der sich **durch** zumindest einen Teil des Körpers erstreckt, wobei der Durchgang zumindest eine proximale Öffnung zum Aufnehmen eines Einführinstruments aufweist; wobei der Durchgang von der Mitte des Körpers beabstandet angeordnet ist, so dass die Torsion während des Einführens der Vorrichtung verringert ist, wobei sich der zumindest eine Durchgang **durch** einen Hauptteil des Körpers erstreckt.

2. Vorrichtung nach Anspruch 1, wobei der Körper der Vorrichtung im Wesentlichen zylindrisch ist.

3. Vorrichtung nach Anspruch 1, wobei der Körper der Vorrichtung verjüngt ist.

4. Vorrichtung nach Anspruch 1, wobei der zumindest eine Durchgang nicht verjüngt ist.

5. Vorrichtung nach Anspruch 1, wobei der zumindest eine Durchgang verjüngt ist.

6. Vorrichtung nach Anspruch 1, wobei der Körper der Vorrichtung zumindest einen Bereich ohne Gewinde umfasst.

7. Vorrichtung nach Anspruch 1, wobei die Vorrichtung aus einem biokompatiblen Material aufgebaut ist.

8. Vorrichtung nach Anspruch 1 bis 7, wobei die Vorrichtung aus Titan, Magnesium, Thallium, rostfreiem Stahl, Nitinol oder einem anderen geeigneten Metall gefertigt ist.

9. Vorrichtung nach Anspruch 1 bis 7, wobei die Vorrichtung aus einem Polymer gefertigt ist.

10. Vorrichtung nach Anspruch 9, wobei die Vorrichtung aus einem biologisch abbaubaren Polymer gefertigt ist.

11. Vorrichtung nach Anspruch 10, wobei die Vorrichtung aus Polysaccharid, Polylactid, Polyglycolid, Polydiaxanol oder anderen geeigneten, biologisch abbaubaren Polymeren oder entsprechenden Copolymeren oder Mischungen gefertigt ist.

12. Vorrichtung nach Anspruch 10, wobei das Material der Vorrichtung eine oder mehrere Zusammensetzungen, ausgewählt aus der Gruppe der Antibiotika, Immunosuppressiva, entzündungshemmenden Medikamente, Knochenwachstumsfaktoren oder anderer geeigneter Pharmazeutika, enthält.

13. Vorrichtung nach Anspruch 1 bis 7, wobei die Vorrichtung mit einer biologisch abbaubaren Beschichtung bedeckt ist.

14. Vorrichtung nach Anspruch 13, wobei die Beschichtung eine oder mehrere Zusammensetzungen, ausgewählt aus der Gruppe der Antibiotika, Immunosuppressiva, entzündungshemmenden Medikamente, Knochenwachstumsfaktoren oder anderer geeigneter Pharmazeutika, enthält.

15. Vorrichtung nach Anspruch 1 bis 7, wobei die Vorrichtung mit einer Beschichtung bedeckt ist, die einen niedrigen Reibungskoeffizienten aufweist.

16. Vorrichtung nach Anspruch 1 bis 7, wobei das proximale Ende einen proximalen Bereich ohne Gewinde umfasst.

17. Vorrichtung nach Anspruch 1 bis 7, wobei die Außengewinde der Vorrichtung selbstschneidend sind.

18. Vorrichtung nach Anspruch 1 bis 7, wobei die Vorrichtung eine Einrichtung zum Aufnehmen eines Gewindes umfasst.

19. Vorrichtung nach Anspruch 1, wobei die zumindest eine proximale Öffnung wie ein Schlitz, ein Kreis oder ein Rechteck geformt ist.

20. Vorrichtung nach Anspruch 1, wobei die Vorrichtung mit dem Instrument über Reibung verbunden ist.

21. Instrument zum Einführen einer Vorrichtung nach Anspruch 1 in ein Gewebe oder eine Struktur mit einer Einrichtung zum Befestigen der zumindest einen proximalen Öffnung und/oder des zumindest einen Durchgangs der Vorrichtung.

22. Instrument nach Anspruch 21, wobei das Instrument als Gewindeschneider arbeitet.

23. Verfahren zur Herstellung einer Vorrichtung nach Anspruch 9 oder 10 unter Verwendung von Spritzgießen.

24. Verfahren zur Herstellung einer Vorrichtung nach Anspruch 9 oder 10 unter Verwendung eines Extrusionsverfahrens in Kombination mit mechanischer Endbearbeitung.

25. Verfahren zur Herstellung einer Vorrichtung nach Anspruch 9 oder 10 unter Verwendung eines Gussblocks, der nachfolgend maschinell bearbeitet wird.

26. Verfahren zur Herstellung einer Vorrichtung nach Anspruch 9 oder 10 unter Verwendung eines durch Polymerisation hergestellten Blocks, der nachfolgend maschinell bearbeitet wird.

27. Verfahren zur Herstellung einer Vorrichtung nach Anspruch 12 oder 14, wobei die Zusammensetzung in die Vorrichtung integriert wird, indem die Vorrichtung mit einer Lösung der Zusammensetzung getränkt wird.

28. Verfahren zur Herstellung einer Vorrichtung nach Anspruch 12 oder 14, wobei die Zusammensetzung vor dem Herstellungsvorgang der Vorrichtung zum Polymer hinzugefügt wird.

29. Verfahren nach Anspruch 23, wobei der Herstellungsvorgang zu einer Ausrichtung des Polymers führt, so dass die Vorrichtung sich nach dem Implantieren entspannt und sich deshalb verkürzt und anschwillt, was zu einer zusätzlichen Verankerung der Vorrichtung in dem Gewebe oder der Struktur führt.

30. Verfahren zum Einführen der Gewindevorrichtung nach Anspruch 1 bis 20 in ein Gewebe oder eine Struktur, umfassend die Schritte:
- Vorsehen einer Vorrichtung mit einem Körper mit einem proximalen Ende und einem distalen Ende, einem Gewindebereich angrenzend an den proximalen und distalen Bereich und so angeordnet, dass er zwischen diesen liegt, und zumindest einem Durchgang, der sich durch einen Hauptteil des Körpers erstreckt, wobei der Durchgang zumindest eine proximale Öffnung zum Aufnehmen des Einführinstruments aufweist; wobei der Durchgang nicht zentriert ist, so dass die Torsion während des Einführens der Vorrichtung durch das Einführinstrument verringert ist,
- schließlich Bohren eines Lochs in das Gewebe oder die Struktur,
- Einführen der Vorrichtung in das Gewebe oder die Struktur über ein Einführinstrument mit verringerter Torsion der Vorrichtung.

## Revendications

1. Dispositif à insérer dans un tissu ou une structure, comprenant :
- un corps ayant une extrémité proximale, une extrémité distale et un centre ; et
- une partie filetée adjacente aux parties proximale et distale et placée de manière à se trouver entre les parties proximale et distale, comportant des filets extérieurs servant à insérer et retenir ledit corps dans le tissu ou la structure,
**caractérisé en ce que**
au moins un passage s'étend à travers au moins une partie du corps, ledit passage ayant au moins une ouverture proximale destinée à recevoir un instrument d'insertion ; le passage étant placé à distance du centre du corps de façon que la torsion pendant l'insertion du dispositif soit réduite, le/les passages s'étendant sur une majeure partie du corps.

2. Dispositif selon la revendication 1, dans lequel le corps du dispositif est sensiblement cylindrique.

3. Dispositif selon la revendication 1, dans lequel le corps du dispositif est conique.

4. Dispositif selon la revendication 1, dans lequel le/les passages ne sont pas coniques.

5. Dispositif selon la revendication 1, dans lequel le/les passages sont coniques.

6. Dispositif selon la revendication 1, dans lequel le corps du dispositif comporte au moins une partie non filetée.

7. Dispositif selon la revendication 1, dans lequel le dispositif est réalisé en matière biocompatible.

8. Dispositif selon les revendications 1 à 7, le dispositif étant réalisé en titane, magnésium, thallium, acier inoxydable, Nitinol ou n'importe quels autres métaux adéquats.

9. Dispositif selon les revendications 1 à 7, le dispositif étant réalisé en polymère.

10. Dispositif selon la revendication 9, le dispositif étant réalisé en polymère biodégradable.

11. Dispositif selon la revendication 10, le dispositif étant réalisé en polysaccharide, polylactide, polyglycolide, polydiaxanole ou autres polymères biodégradables adéquats ou en copolymères ou mélanges appropriés.

12. Dispositif selon la revendication 10, dans lequel la matière du dispositif contient un ou plusieurs composés choisis dans le groupe comprenant les antibiotiques, les immunosuppresseurs, les anti-inflammatoires, des facteurs de croissance osseuse ou autres substances pharmaceutiques adéquates.

13. Dispositif selon les revendications 1 à 7, le dispositif étant couvert par un revêtement biodégradable.

14. Dispositif selon la revendication 13, dans lequel ledit revêtement contient un ou plusieurs composés choisis parmi le groupe comprenant les antibiotiques, les immunosuppresseurs, les anti-inflammatoires, les facteurs de croissance osseuse ou autres substances pharmaceutiques adéquates.

15. Dispositif selon les revendications 1 à 7, le dispositif étant couvert par un revêtement à faible coefficient de frottement.

16. Dispositif selon les revendications 1 à 7, dans lequel l'extrémité proximale comporte une partie proximale non filetée.

17. Dispositif selon les revendications 1 à 7, dans lequel lesdits filets extérieurs du dispositif sont autotaraudeurs.

18. Dispositif selon les revendications 1 à 7, ledit dispositif comportant un moyen destiné à recevoir un filetage.

19. Dispositif selon la revendication 1, dans lequel le/les ouvertures proximales se présentent sous la forme d'une fente, d'un cercle ou d'un carré.

20. Dispositif selon la revendication 1, dans lequel le dispositif se fixe à l'instrument par frottement.

21. Instrument pour insérer un dispositif selon la revendication 1 dans un tissu ou une structure, comprenant un moyen destiné à se fixer à/aux ouvertures proximales et/ou à/aux passages du dispositif.

22. Instrument selon la revendication 21, l'instrument servant de fraise de filetage.

23. Procédé pour fabriquer, par moulage par injection, un dispositif selon la revendication 9 ou 10.

24. Procédé pour fabriquer un dispositif selon la revendication 9 ou 10, par extrusion combinée avec un finissage mécanique.

25. Procédé de fabrication d'un dispositif selon la revendication 9 ou 10 à l'aide d'un bloc moulé, qui est ensuite usiné.

26. Procédé de fabrication d'un dispositif selon la revendication 9 ou 10 à l'aide d'un bloc obtenu par polymérisation, qui est ensuite usiné.

27. Procédé de fabrication d'un dispositif selon la revendication 12 ou 14, dans lequel le composé est incorporé dans le dispositif par incubation d'une solution du composé dans le dispositif.

28. Procédé de fabrication d'un dispositif selon la revendication 12 ou 14, dans lequel le composé est ajouté au polymère avant le processus de fabrication du dispositif.

29. Procédé selon la revendication 23, le procédé de fabrication provoquant une orientation du polymère de façon que le dispositif se relâche après l'implantation et, par conséquent, qu'il se contracte et qu'il se déploie, ce qui provoque un ancrage supplémentaire du dispositif dans le tissu ou la structure.

30. Procédé pour insérer le dispositif fileté selon les revendications 1 à 20 dans un tissu ou une structure, comprenant les étapes consistant à :
- réaliser un dispositif comprenant un corps ayant une extrémité proximale et une extrémité distale, une partie filetée adjacente aux parties proximale et distale et placée de façon à se trouver entre les parties proximale et distale et au moins un passage s'étendant sur une majeure partie du corps, ledit passage ayant au moins une ouverture proximale pour recevoir un instrument d'insertion ; le passage étant non centré de façon que la torsion soit réduite pendant l'insertion du dispositif par l'instrument d'insertion ;
- percer finalement un trou dans le tissu ou la structure ; et
- insérer le dispositif dans le tissu ou la structure à l'aide d'un instrument d'insertion à torsion réduite du dispositif.
